# EUROPEAN PATENT APPLICATION

(11) **EP 3 147 664 A1**
(43) Date of publication of application: **29.03.2017**
(21) Application number: 15306470.4
(22) Date of filing: 22.09.2015
(51) Int. Cl.: G01N 33/574, G06F 19/00

(54) **A SCORING METHOD FOR PREDICTING THE EFFICIENCY OF A TREATMENT WITH ANTI-PD-1 AND/OR ANTI-PD-L1 MONOCLONAL ANTIBODIES**

(71) Applicant: Institut Gustave Roussy, 94805 Villejuif Cédex (FR)
(72) Inventor: GIRAULT, Isabelle, 94800 Villejuif (FR); VAGNER, Stephan, 91370 Verrières le Buisson (FR); ROBERT, Caroline, 92260 Fontenay aux Roses (FR); LANOY, Emilie, 75013 Paris (FR); TEXIER, Matthieu, 92160 Antony (FR); ADAM, Julien, 92500 Rueil-Malmaison (FR); SHEN, Shensi, 75014 Paris (FR)
(74) Representative: Cabinet Plasseraud

(57) **Abstract**

The invention aims at a method implemented by computer means (CC) for assessing an efficiency of a cure of a cancer disease based on at least one antibody against protein PD-1 and/or its ligand PD-L1, wherein the method comprises the steps:
- Obtaining data of at least one fluorescence microscope (MIC) image (IM DAT) of cells of a tumor material (TAM) treated for performing a test of Proximity Ligation Assay to assess an interaction between protein PD-1 and its ligand PD-L1 in at least some of said cells,
- Counting in said image a number of spots related to said interactions between PD-1 and PD-L1, and a number of nuclei in said image, and
- Estimating a ratio between said number of spots and said number of nuclei with a view to assess an efficiency of a cure based on at least one antibody against protein PD-1 and/or its ligand PD-L1 on a cancer disease from which said tumor material results.

## Description

The present invention relates to a computer implemented method for assessing the efficiency of a treatment based on anti-PD-1 and/or anti-PD-L1 monoclonal antibodies.

Immunotherapy has been revolutionized by the concept of breaking tolerance. It represents a major paradigm shift that marks the beginning of a new era in treatment of cancer diseases. The impact of the first checkpoint inhibitors, named "anti-CTLA-4" (cytotoxic T lymphocyte antigen-4) and "anti-PD-1/ anti-PD-L1" (programmed death-1 receptor and its ligand, PD-L1) is unprecedented. In the recent years, advanced melanoma has been transformed from an incurable disease into a potentially curable disease and it is believed to have also a transversal impact throughout solid tumor oncology.

In advanced melanoma, response rates are about 12% for anti-CTLA-4 and about 40% for anti-PD-1, and are remarkably durable, hence their impact on survival.

In melanoma, anti-CTLA-4 (ipilimumab™) was approved in 2011 and anti-PD-1 (pembrolimumab™) in 2014. Another anti-PD-1 antibody (nivolumab™) has been recently approved based on phase III trial results in metastatic melanoma without BRAF mutation. Ipilimumab™ already has been evaluated in the adjuvant setting (European Organization for Research and Treatment of Cancer [EORTC] 18071) and shown to significantly improve recurrence-free survival in stage III patients at high risk of relapse. An adjuvant trial to evaluate pembrolizumab™ in this population (EORTC 1325) was started in early 2015.

Treatments with anti-PD-1 or anti-PD-L1 monoclonal antibodies are associated with clinical response in patients with melanoma. 30 to 40% of response rates have been achieved with pembrolizumab™ and nivolumab™ (which are already on the market in the United States of America for patients with metastatic melanoma) as well as promising clinical results in patients with other tumor types: lung cancers, renal cell cancer, Hodgkin lymphomas, gastric cancers, etc.

Statistics have thus shown globally that some patients could have been cured thanks to this kind of treatment. Yet, in some cases, no positive evolution could have been observed. However, this kind of treatment can be expensive and can have further unwanted side effects.

A characterization technique is therefore needed to assess an efficiency of that treatment. Some attempts of characterization are listed below.

Regarding PD-1 and PD-L1 as biomarkers to be used for that characterization, although the number of intra-tumoral PD-1 positive-cells before treatment of patients with melanoma with anti-PD-1 mAb pembrolizumab™ (mAb standing for "monoclonal antibody therapy") is higher in responder patients than in patients who progress under therapy in a series of 46 patients, PD-1 positive cells at baseline has not been found to be a robust predictive marker for response to treatment.

Expression of PD-L1 is one of the biomarkers being associated with a higher level of response rate to these immunotherapies. However, expression of PD-L1 alone is neither a very specific, nor sensitive, biomarker. First, PD-L1 alone is not a stable marker as it can be found positive and negative at the same time in various metastases from the same patient. Second, some patients with negative PD-L1 expressing melanoma can have excellent and durable antitumor responses.

In a recent publication, the presence of CD8 positive lymphocytes at the margin of the tumors was a more potent predictive biomarker for efficacy of anti-PD-1 pembrolizumab™ than PD-L1 expression or PD-1 expression (Tumeh et al, *Nature,* 2014).

However, although they are neither sensitive, nor specific enough, to serve as biomarkers on a routine base, the presence of cells expressing PD-1 and PD-L1 together with the presence of proliferating CD8 T cells in the margin of and inside the tumor are associated with clinical responses in patients with metastatic melanoma.

In patients with lung cancer, the association between PD-L1 expression in the tumor cells as well as in the tumor stroma seems more associated with responses to anti-PD-1 and anti-PD-L1 drugs than in melanoma patients.

Altogether, expression of PD-L1 alone is not sufficient to select patients for response to anti-PD-1 or anti-PD-L1 therapies.

Additional biomarkers are needed.

One reason also why PD-L1 expression alone might not be specific biomarker might be that its expression can be induced by various stimuli (including intrinsic PI3K signaling pathway, via a PTEN mutation for example), whereas, it is more likely that its predictive value might be stronger when its expression is induced by the presence of cytokine secreting T cells in the microenvironment.

Indeed, if there is no PD-1-expressing T cell around, it is quite unlikely that PD-L1 can serve as a predictive biomarker.

Furthermore, there is no consensus on the type of antibodies, neither on the method of staining, nor on the threshold for positivity for immuno-staining with anti-PD-L1 antibodies. For example, for melanoma, a threshold for positivity of 1% of tumor cells is proposed, while that threshold is 5% with distinct antibodies. Some studies consider immune cells and not only tumor cells, whereas, other studies take only tumor cells into account.

This illustrates why PD-L1 immuno-staining cannot be considered as a robust biomarker to predict response to treatment.

PD-L2 is another ligand for PD-1 but there is no known standardized and reliable way to perform immuno-staining for PD-L2 expression.

The present invention aims to improve this situation.

To that end, the invention proposes a method implemented by computer means for assessing an efficiency of a cure of a cancer disease based on at least one antibody against protein PD-1 and/or its ligand PD-L1, wherein the method comprises the steps:
- Obtaining data of at least one fluorescence microscope image of cells of a tumor material treated for performing a test of Proximity Ligation Assay (PLA) to assess an interaction between protein PD-1 and its ligand PD-L1 in at least some of said cells,
- Counting in said image a number of spots related to said interactions between PD-1 and PD-L1, and a number of nuclei in said image, and
- Estimating a ratio between said number of spots and said number of nuclei with a view to assess an efficiency of a cure based on at least one antibody against protein PD-1 and/or its ligand PD-L1 on a cancer disease from which said tumor material results.

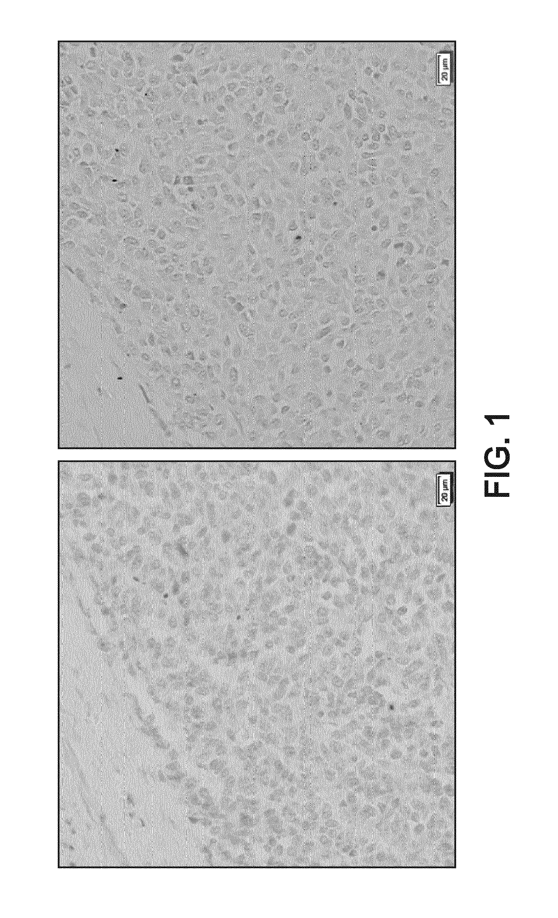

As detailed below with reference to figures 1 to 5, it appeared that PLA is much more robust than simple PD-1 and PD-L1 co-staining, yielding then a new characterization method for assessing the efficiency of a treatment based on PD-1 and/or PD-L1 antibodies.

In an embodiment, said tumor material is an infiltrate. That embodiment shows good statistical characterization results.

In another possible embodiment, said tumor material is a tumor tissue.

In still another possible embodiment, said tumor material includes both an infiltrate and a tumor tissue. That embodiment shows also very good statistical characterization results.

In an embodiment, a TMB chromogenic revelation is applied to at least tissues of said tumor material (where TMB stands for "3,3',5,5'-Tetramethylbenzidine"). This embodiment enables the PLA observation of pigmented tumor material, including typically melanin tissues.

The method according to an embodiment of the invention further comprises a previous step wherein a statistical predictive model is built at first on the basis of:
* observations on patients having an antibody PD-1 and/or PD-L1 treatment, and being:
   - in response to the treatment, assessing thus an efficiency of the treatment, or
   - still in progression, assessing thus an inefficiency,
* and measurements of said ratio based on PLA tests applied to a tumor material of said patients.

More particularly, in that embodiment, predictive values of said ratio measurements can be assessed in measuring for example the following parameters:
- A proportion of true positives among responders,
- A proportion of true negatives among progressors,
- A proportion of responders among predicted positives, and
- A proportion of progressors among predicted negative.

Therefore, based on that predictive model, assessment of how a patient would react to said treatment can be given according to PLA tests applied on a tumor material of that patient. Indeed, once the predictive model is built, the predictive model can be applied to PLA test data (ratio measurements of spots number/nuclei number), such PLA tests being performed on a tumor material of a current patient, so as to determine whether that current patient would respond to the treatment or, on the opposite, would be still in progression.

Therefore, in a general embodiment data of said estimated ratio are collected from said tumor material and probabilities are estimated on the basis of said collected data to forecast:
- a response to the treatment, or
- on the opposite, a progression
of a patient having a PD-1 and/or PD-L1 antibody based treatment.

As explained above, these probabilities can be estimated on the basis of a predictive model applied on said collected data.

The present invention aims also at a drug comprising at least one antibody against PD-1 and/or its ligand, for use in a method of treating cancer in a patient, wherein the patient has been selected to have probabilities to be in response to said drug of higher or equal to a predetermined threshold (for example 50%), said probabilities being determined as defined above.

The present invention aims further at a computer program comprising instructions for performing the method exposed above, when run by a processor.

The present invention further aims at a device comprising a computer circuit connected to a camera acquiring at least one fluorescence microscope image of cells of a tumor material treated for performing a test of Proximity Ligation Assay (PLA), said computer circuit being arranged to perform the method exposed above.

The invention proposes therefore a visualization of the interaction between PD-1 and its ligand PD-L1, as a molecular approach.

According to the invention, a proximity ligation assay (PLA) that specifically stains the individual interaction between these two molecules is used so as to visualize the PD-L1 molecules that were engaged in an interaction with PD-1.

As an advantage of the invention, the PD-1/PD-L1 PLA gives a much cleaner image than the double staining with both antibodies, so as to show co-localization of the two molecules. Using the PLA technology, each PD-1 molecule that is in close vicinity with its ligand PD-L1 can be visualized as a well-defined colored dot. The usual distance for interaction between the two molecules is in the range between 5 and 20 nm: if such a distance is found between the two molecules and merely in a same 3D plane, then an interaction can be assessed.

The present invention is illustrated by way of example, and not by way of limitation, in the figures of the accompanying drawings, in which like reference numerals refer to similar elements and in which:
- Figures 1 to 5 show PD-1 (in black) and PD-L1 (in white) co-staining in a tumor material image (left side of the figures), to be compared with a PLA test performed on a same tumor material (black spots evidencing a co-localization between PD-1 and PD-L1);
- Figure 6 shows an exemplary embodiment of steps of a method according to the invention;
- Figure 7 shows an exemplary embodiment of a device according to the invention.

Proximity Ligation Assay ("in situ PLA") is a technique that extends the capabilities of traditional immunoassays to include direct detection of proteins, protein interactions and modifications with high specificity and sensitivity. Protein targets can be readily detected and localized with single molecule resolution and objectively quantified in unmodified cells and tissues. The left parts of figures 1 to 5 shows individual molecules PD-1 (in black) and PD-L1 (in white) around nuclei (in light grey). Utilizing only a few cells, sub-cellular events, even transient or weak interactions, are revealed in situ and sub-populations of cells can be differentiated. Within hours, results from conventional co-immuno-precipitation and co-localization techniques can be confirmed. The right parts of figures 1 to 5 shows such a co-localization between PD-1 and PD-L1 molecules (a black spot for each occurrence of co-localization) around same cells (shown in light grey).

According to the PLA principle, two primary antibodies raised in different species recognize the target antigen or antigens of interest. Species-specific secondary antibodies ("PLA probes"), each with a unique short DNA strand attached to it, bind to the primary antibodies. When the PLA probes are in close proximity, the DNA strands can interact through a subsequent addition of two other circle-forming DNA oligonucleotides. After joining of the two added oligonucleotides by enzymatic ligation, they can be amplified via for example rolling circle amplification using a polymerase. After the amplification reaction, several-hundredfold replication of the DNA circle has occurred, and labeled complementary oligonucleotide probes highlight the product. The resulting high concentration of fluorescence in each single-molecule amplification product is then easily visible as a distinct spot when viewed with a fluorescence microscope (black spots on the right sides of figures 1 to 5).

### Description of an example of embodiment

In an example of embodiment given hereafter, Proximity Ligation Assays (PLAs) were performed on melanoma tissue sections. Following dewaxing and rehydrating of tissue sections, antigen retrieval was performed by heating the slides for 45 minutes at 95°C in citrate buffer, with a pH around 6. The PLA protocol was followed according to manufacturers' instructions (OLINK Bioscience™, Uppsala, Sweden), with incubation of the primary antibodies at 4°C overnight. After blocking (OLINK), the antibodies were used at the following concentrations: for PD-1 (mouse, ab52587, Abcam 1:300) and for PD-L1 (rabbit, 13684; Cell Signalling 1:400). PLA minus and plus probes (containing the secondary antibodies conjugated with oligonucleotides) were added and incubated during one hour at 37°C. Afterwards, further oligonucleotides were added, allowed to hybridise to the PLA probes, and ligase joined the two hybridised oligonucleotides to a closed circle. The DNA was then amplified (rolling circle amplification), and detection of the amplicons was carried out using a TMB chromogenic revelation in blue with a counterstain in Fast red. Results can be visualized by a scanner Olympus™ VS120.

Automated immuno-histo-chemistry on Ventana Discovery Ultra™ platform-epitope retrieval in CC1 buffer is performed during 92 minutes at 95°C. A first staining with anti-PD-L1 antibody, revelation kit anti-rabbit amplification-HQ, enzyme HRP, chromogen DAB, and denaturation is performed with a heating during 8 minutes at 90°C, using a revelation kit. A second staining with anti-PD-1 antibody, revelation kit anti-mouse UltraMAP with amplification, enzyme HRP, chromogen Discovery purple, is then carried out.

### Preferable options

A first preferred step is the choice of the antibodies for each protein. In order to obtain a reliable and reproductive result, three different antibodies have been tested for anti-PD-1 and six for anti-PD-L1 in three different antigen retrieval protocols.

The integrity of the target complex in tissue samples is a second challenge. PD-1 and PD-L1 are expressed at the membrane of distinct types of cells. Freshly cut tissue sections only have to be used, and the slides must not be exposed to extreme temperature orders to preserve the structure of the cell membranes.

Figures 1 to 5 have been obtained on melanin tissues. Care had to be taken in order to differentiate in between dark spots (assessing an interaction between the two molecules of interest) and the melanin tissues, present in melanoma cells (which are physiologically pigmented). To do so, the detection method had to be modified, and, instead of using DAB tags for the detection of the amplicons, TMB chromogenic revelation in blue with a counterstain in Fast red were applied to the tissue samples.

### Co-staining PD-1 and PD-L1 versus PLA test

The co-staining PD-1 and PD-L1 can visualize both PD-1 and PD-L1 molecules and roughly estimate the distance between them. However, because the tissue section has a sizeable thickness (practically around several micrometers), false positive due to superposition can occur (left sides of figures 1 to 5). When the PLA test is positive, it means by definition that the two molecules are in close vicinity, at a maximal distance of 20 nm one from another (dark spots in right sides of figures 1 to 5).

The PLA characterization appears therefore much more robust than co-staining or any other method of the prior art. Yet, co-staining would be the very natural approach for any man skilled in the art.

Figures 1 to 5 show a comparison between PD-1 and PD-L1 co-localisation by immunohistochemistry (IHC) in melanoma (left side of the figures) and PD-1/PD-L1 PLA tests performed thereon (right side).

For all figures 1 to 5, the PD-1 staining is in DAB (usually brown but tinted in black for the sake of clarity in the left sides of the figures) and the PD-L1 (usual staining in purple, but tinted in white in the left sides of the figures). For the same tumor, a PLA test on PD-1/PD-L1 is performed and each black dot in the right sides of the figures 1 to 5 represents the two proteins in close proximity.

Figure 1 shows a negative staining in a tumoral sample. IHC is negative (no PD-1 and no PD-L1) and PLA is consequently negative also (no black spot). The patient is in progression.

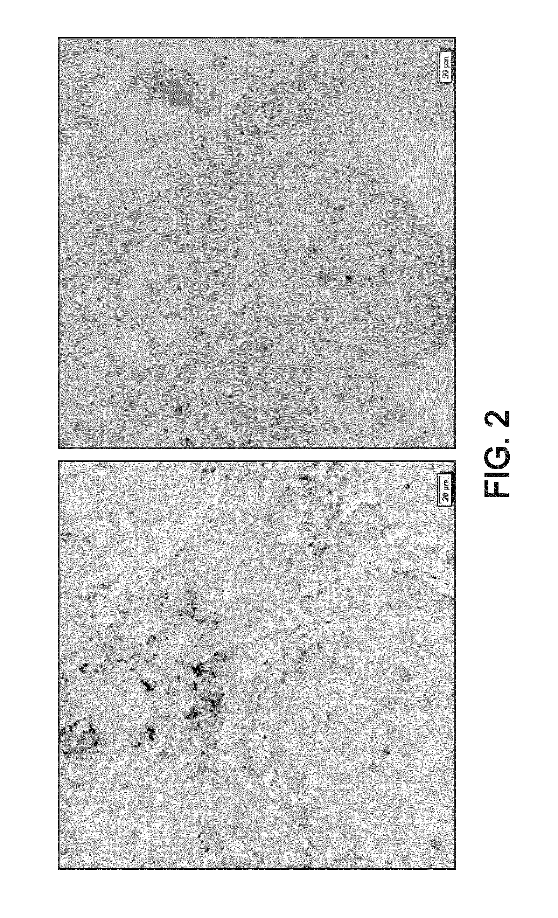

Figure 2 shows a weak staining in infiltrate (from the upper left corner to the lower right corner) and no staining in tumoral sample. PD-1 IHC staining is weak and PD-L1 IHC staining is very weak. The conclusion about the possible interaction between the two proteins is not obvious with these staining. The PLA staining shows few dots and the predicted response is then easier to determine, than the PD-1 and PD-L1 co-staining taken alone.

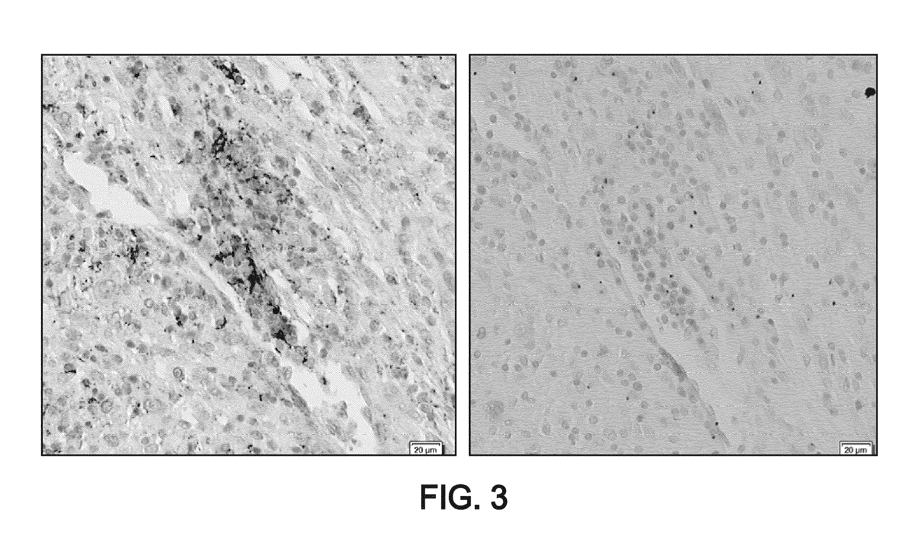

Figure 3 shows a weak staining in infiltrate and no staining in tumoral sample. PD-1 IHC staining is weak and PD-L1 IHC staining is very weak. The conclusion about the possible interaction between the two proteins is not obvious with these staining. The PLA staining shows few dots and the predicted response is easier to determine than the PD-1 and PD-L1 staining.

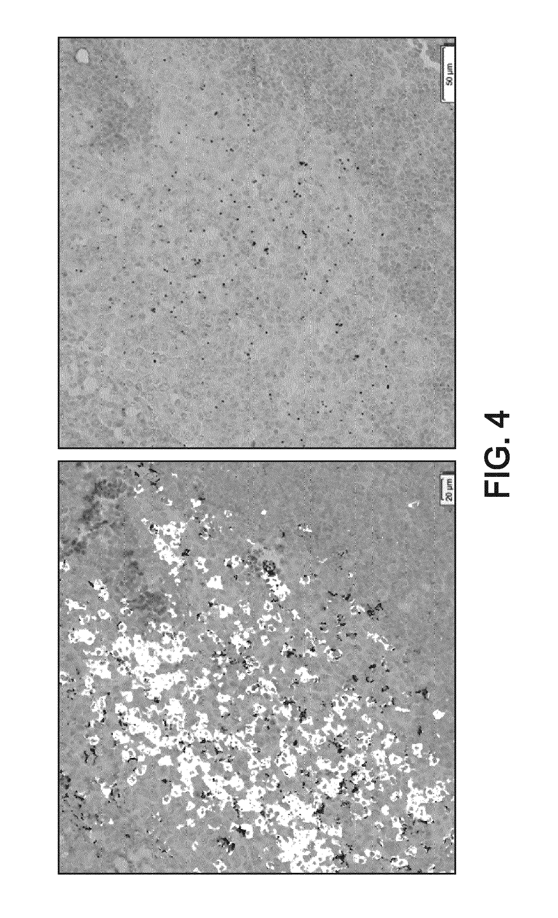

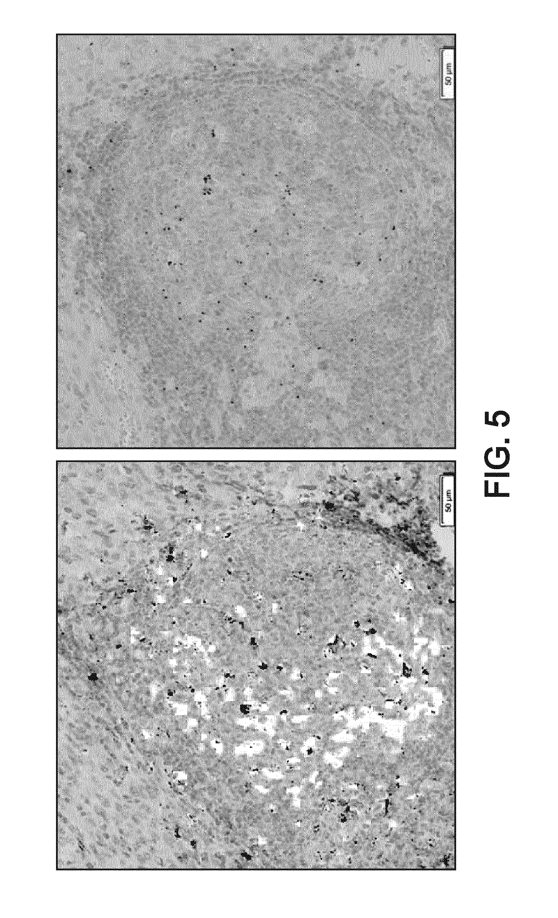

Figures 4 and 5 show a staining in the germinal center in a lymph node. PD-1 and PD-L1 IHC staining are both strong. The PLA test shows also several dots. Lymph node is then the place of recruitment of both cells PD-1+ and PD-L1+. Lymph nodes are then a good example of application of the invention.

For all tissue section, in a digital image as shown in the right part of figures 1 to 5, a total number of PLA spots (NS) and a total number of nuclei (NN) are evaluated in the total infiltrate. To that end, an image contrast optimizer and shape recognition software (Hough transformation) can be used for identifying the nuclei and the PLA spots, and for counting them.

The results of the ratio NS/NN (spots/nuclei) are given per regions in each image:
- for the tumors only, without infiltrate,
- for the infiltrate only, and
- for the tumors with infiltrate.

### Statistical results

The association between the PD-1/PD-L1 staining assessed by PLA and the response to anti-PD-1/anti-PD-L1 therapy was evaluated to assess the predictive value of the PD-1/PD-L1 staining.

The primary endpoint was the observed clinical response to anti-PD-1/anti-PD-L1 therapy.

The effects of the following biomarkers as continuous variables were assessed:
- Number of spots per nucleus in infiltrate only,
- Number of spots per nucleus in tumor only,
- Number of spots per nucleus in both tumor and infiltrate.

Probability for each patient to respond to anti-PD-1/anti-PD-L1 therapy was derived from univariable logistic regression (one biomarker) and from multivariable logistic regression (both the three biomarkers).

From the staining results as biomarker, the model classified patients as responders and non-responders: when the staining results corresponded to a predicted probability of clinical response over 50%, patients were classified as predicted responders ; when the staining results corresponded to predicted probability of clinical response below 50%, patients were classified as predicted non-responders.

### Table 0 Predicted and observed clinical responses

| | | Observed clinical response | |
|---|---|---|---|
| Predicted clinical response | | + Responder | - Progressor |
| | + | True Positives | False Positives |
| | - | False Negatives | True Negatives |

Predictive value of the biomarkers was assessed in measuring the following parameters:
- Sensitivity (Se): proportion of true positives among responders,
- Specificity (Sp): proportion of true negatives among progressors,
- Positive predictive value (PPV): proportion of responders among predicted positives,
- Negative predictive value (NPV): proportion of progressors among predicted negative.

Exact confidence intervals for each parameter were computed using Clopper-Pearson method. Akaike information criteria (AIC) from the different models were compared to determine the best model (the one with the lowest AIC).

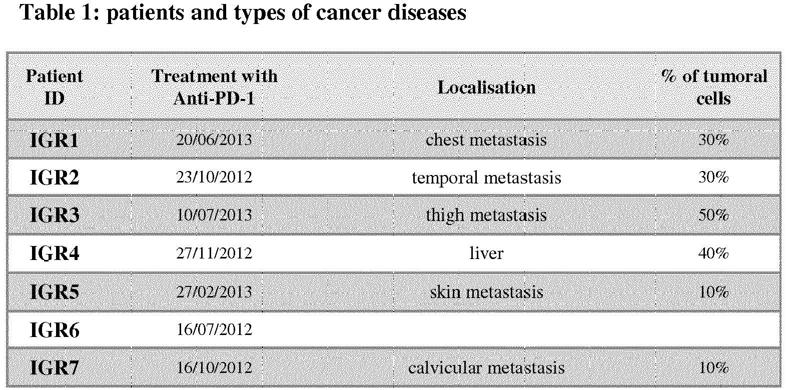

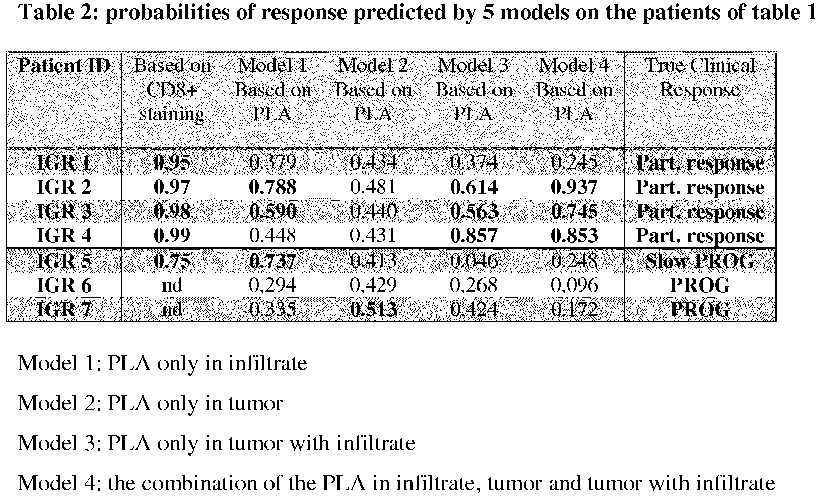

Patients IGR 1 to 4 are in partial response, while patients IGR 5 to 7 are in progression.

The model based on staining only is weaker when compared to the PLA models. It can be observed further that the model using PLA in tumor only seems to show weaknesses when compared to the other PLA models. Referring to table 3, a preferred model in the given example is Model 3 (having further the lowest AIC).

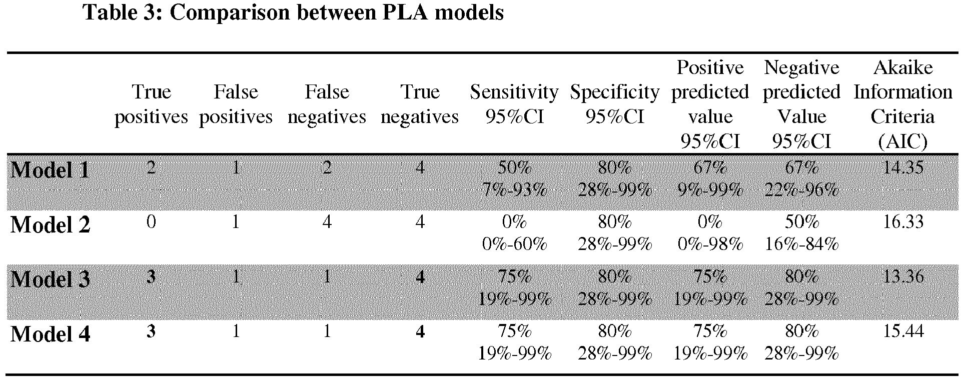

### General method

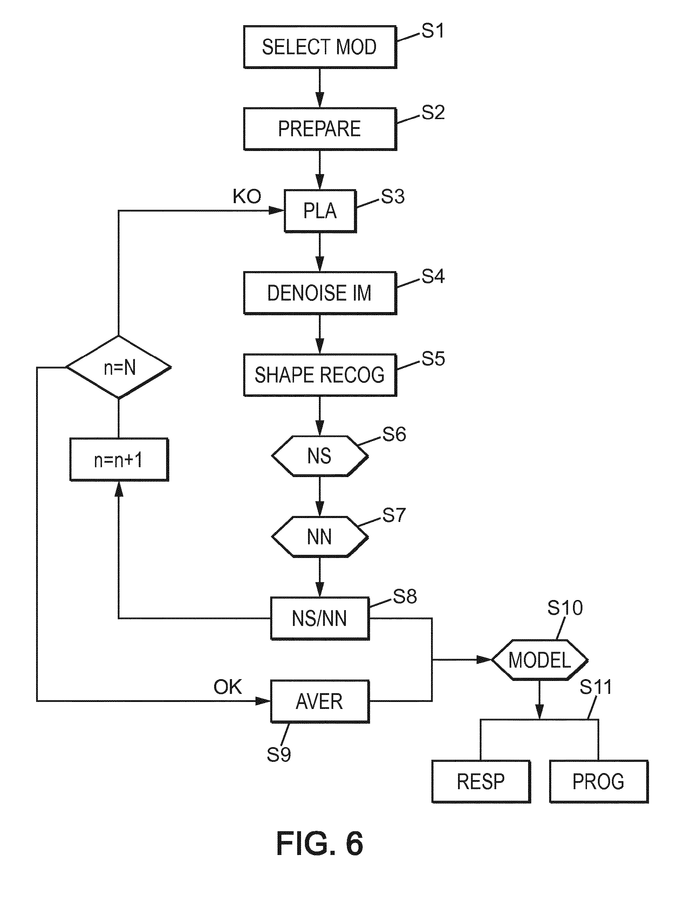

Referring now to figure 6, an example of a method according to the invention comprises a first step S1 of selecting, from a tumor material previously extracted from the body of a patient, either:
- The infiltrate of the material,
- The tumor tissue of the material, or
- Both the infiltrate and the tumor tissue,
to perform a chosen model among the four models using PLA characterization as described above.

The choice of one model among the others may depend on the cancer type (lung, melanoma, liver, etc.).

Hence, once the selected tumor material and the corresponding model are chosen in step S1, the tumor material is prepared in step S2 (IHC) so as to perform in step S3 the PLA test. A PLA digital image is then obtained and a subsequent step S4 consists on applying software treatment so as to enhance contrast in the image and, in step S5, recognize in it dark spots and nuclei (for example with a shape recognition algorithm). In step S6, the dark spots showing co-localization are counted (NS) and the number of nuclei is further counted (NN) in step S7, so as to calculate the ratio between NS and NN in step S8. Of course, steps S3 to S8 can be repeated several times (N) so as to estimate for example an average of the measurement of the ration NS/NN in step S9. The ratio values and/or the average value are inputted in step S10 in the chosen model to determine in step S11 whether the patient is significantly in response to the treatment or in progression.

More generally, the method can also be applied to determine whether the patient would respond to the treatment, before the application itself of the treatment based on PD-1 and/or PD-L1 antibodies. The PLA test would then help to determine whether the patient would be in response to that kind of treatment or not, and more precisely to what kind of antibodies, and possibly in which conditions and quantities. Therefore, the present invention aims also at a drug, adapted for a treatment which is appropriate to a given patient, and comprising at least one antibody against PD-1 and/or its ligand PD-L1. More particularly, that patient has been selected to have probabilities to be in response to that drug of higher or equal to a predetermined threshold, for example 50% in the examples given above for building statistical models 1 to 4.

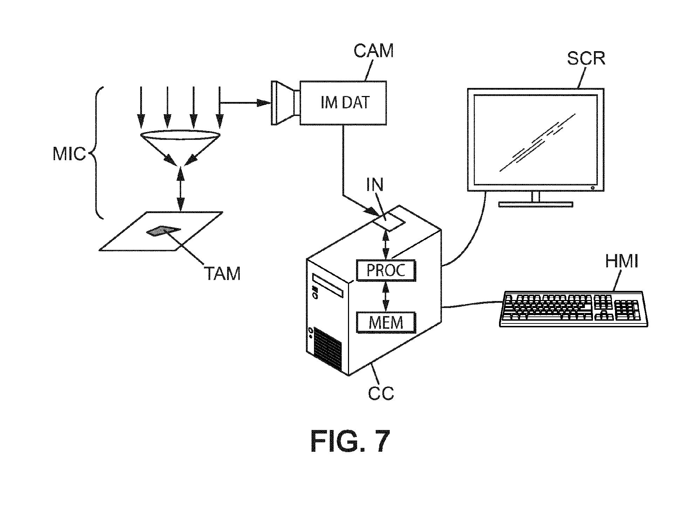

Referring now to figure 7, the device according to the invention can comprise a computer circuit CC connected to a camera CAM acquiring from a microscope MIC fluorescence microscope image data of cells of a tumor material TAM treated for performing a test of Proximity Ligation Assay (PLA). The computer circuit CC includes typically an input interface IN to receive the image data IM DAT, a storage memory unit MEM to store at least transitorily image data in view to process them (as a working memory), and a processor PROC so as to treat the image data according to steps S4 to S11 presented above. More particularly, the memory unit MEM further stores computer program instructions (non-transitorily) to be run by the processor PROC. The algorithm of such a computer program can correspond to steps S4 to S11 commented above. The device can further comprise a screen SCR or a printer to display at least the result of the application of the model in step S11, and also a human-machine interface to control the operation of the device (keyboard, touchscreen, mouse, etc.).

## Claims

1. A method implemented by computer means for assessing an efficiency of a cure of a cancer disease based on at least one antibody against protein PD-1 and/or its ligand PD-L1, wherein the method comprises the steps:
- Obtaining data of at least one fluorescence microscope image of cells of a tumor material treated for performing a test of Proximity Ligation Assay (PLA) to assess an interaction between protein PD-1 and its ligand PD-L1 in at least some of said cells,
- Counting in said image a number of spots related to said interactions between PD-1 and PD-L1, and a number of nuclei in said image, and
- Estimating a ratio between said number of spots and said number of nuclei with a view to assess an efficiency of a cure based on at least one antibody against protein PD-1 and/or its ligand PD-L1 on a cancer disease from which said tumor material results.

2. The method of claim 1, wherein said tumor material is an infiltrate.

3. The method of claim 1, wherein said tumor material is a tumor tissue.

4. The method of claim 1, wherein said tumor material includes both an infiltrate and a tumor tissue.

5. The method according to any of the preceding claims, wherein a TMB chromogenic revelation is applied to said tumor material.

6. The method according to any of the preceding claims, comprising a previous step wherein a statistical predictive model is built at first on the basis of:
* observations on patients having an antibody PD-1 and/or PD-L1 treatment, and being:
- in response to the treatment, assessing thus an efficiency of the treatment, or
- still in progression, assessing thus an inefficiency,
* and measurements of said ratio based on PLA tests applied to a tumor material of said patients.

7. The according to claim 6, wherein predictive values of said ratio measurements are assessed in measuring the following parameters:
• A proportion of true positives among responders,
• A proportion of true negatives among progressors,
• A proportion of responders among predicted positives, and
• A proportion of progressors among predicted negative.

8. The method according to any of the preceding claims, wherein data of said estimated ratio are collected from said tumor material and wherein probabilities are estimated on the basis of said collected data to forecast:
- a response to the treatment, or
- on the opposite, a progression
of a patient having a PD-1 and/or PD-L1 antibody based treatment.

9. The method of claim 8, taken in combination with anyone of claims 6 and 7, wherein said probabilities are estimated on the basis of a predictive model applied on said collected data.

10. Drug comprising at least one antibody against PD-1 and/or its ligand, for use in a method of treating cancer in a patient, wherein the patient has been selected to have probabilities to be in response to said drug of higher or equal to a predetermined threshold, said probabilities being determined in accordance with the method of anyone of claims 8 and 9.

11. A computer program comprising instructions for performing the method of anyone of claims 1 to 9, when run by a processor.

12. A device comprising a computer circuit connected to a camera acquiring at least one fluorescence microscope image of cells of a tumor material treated for performing a test of Proximity Ligation Assay (PLA), wherein said computer circuit is arranged to perform the method according to anyone of claims 1 to 9.
